# EUROPEAN PATENT SPECIFICATION

(11) **EP 1 253 866 B1**
(45) Date of publication and mention of the grant of the patent: **19.07.2006**
(21) Application number: 01904078.1
(22) Date of filing: 05.02.2001
(51) Int. Cl.: A61B 18/14

(54) **AN ELECTROSURGICAL INSTRUMENT AND AN ELECTROSURGERY SYSTEM INCLUDING SUCH AN INSTRUMENT**
EIN ELEKTROCHIRURGISCHES INSTRUMENT UND EIN ELEKTROCHIRURGISCHES SYSTEM MIT EINEM SOLCHEN INSTRUMENT
INSTRUMENT D'ELECTROCHIRURGIE ET SYSTEME D'ELECTROCHIRURGIE COMPRENANT CET INSTRUMENT

(30) Priority: 08.02.2000 GB 0002849
(43) Date of publication of application: 06.11.2002
(73) Proprietor: GYRUS MEDICAL LIMITED, Cardiff CF3 0LT (GB)
(72) Inventor: GOBLE, Colin Charles Owen, Englefield Green, Egham Surrey, TW20 0XE (GB); GOBLE, Nigel Mark, Castleton, Near Cardiff CF3 8SB (GB)
(74) Representative: Blatchford, William Michael
(86) International application number: PCT/GB2001/000466
(87) International publication number: WO 2001/058371

(56) References cited:
- WO-A-99/49799
- US-A- 4 116 198
- US-A- 5 009 656

## Description

This invention relates to an electrosurgical instrument and an electrosurgery system including the instrument and a radio frequency generator system for treatment of tissue, wherein the operative site and distal portion of such an instrument are immersed in an aqueous solution during use to vaporise, coagulate, desiccate or otherwise thermally modify such tissues.

Electrosurgery has been used in surgical practice for over fifty years and during that time has seen a number of improvements to enhance safety and performance in more challenging surgical environments. One such challenge has been the emerging use of minimal access or endoscopic techniques to perform an ever increasing range of surgical procedures.

Traditional monopolar and bipolar electrosurgical devices have been widely used in endoscopic surgery but have suffered from a number of technical disadvantages, particularly when the operative site is distended or irrigated with aqueous solutions. Such solutions are commonly employed to improve endoscopic visualisation and are usually introduced to the body cavity through a specific channel provided in the endoscope itself. The immersion of electrosurgical instruments in aqueous solutions introduces a number of technical hurdles in terms of insulation and preventing power dissipation to the solution rather than the target site.

Monopolar electrosurgery requires the aqueous solution to be non-conductive in order to operate efficiently. Despite use of solutions such as Dextrose. Sorbitol and Glycine which have very low values of electrical conductance, tissue and bodily fluids released during the operation can significantly increase the conductance of fluids bathing the target site. This usually necessitates increasing power output to overcome losses to the solution in order to maintain performance. Increasing power output increases the recognised risk of inadvertent burns to the patient or operator when using monopolar arrangements.

Bums associated with monopolar arrangements can be avoided, albeit in an instrument with limited power, by use of capacitive coupling in a return path between tissue adjacent the operative site and the conductive casing of a self-contained battery-powered instrument such as that disclosed in the applicant's International Application No. WO97/15237. This device has a single exposed electrode and with an integral generator inside a metallic handheld casing and is intended for dry field use at high frequencies. The casing, acts as a conductor capacitively coupled to its surroundings to provide a capacitive return path from tissue being treated to the generator within the casing. The impedance of the return path is widely variable due to the varying degree to which the instrument is capacitively coupled to the surgeon's hand and the variable juxtaposition of surrounding conductive masses. A thin plastics coating is provided over the casing to prevent direct electrical contact with conductive masses.

While a conventional bipolar electrosurgical instrument, in which the two poles of the electrosurgical output are mounted as neighbouring electrodes on the tip of the instrument and both are required to contact tissue in order to produce an effect, to a large extent eliminate the risks of burns, the output power will still preferentially pass through conductive fluid present at the application site rather than the tissue itself. Nonetheless, the heating of such fluid can produce secondary heating of the tissue and thereby produce coagulation or desiccation of the tissue. The problem is much more evident when trying to use a bipolar arrangement to cut or ablate tissue whereby the solution lowers the impedance of the output and prevents the output voltage reaching the levels necessary to induce vaporisation of the tissue, typically requiring a peak-to-peak voltage in excess of 500V.

U.S. Patent No. 5,009,656, Reimels, describes a method of overcoming this problem in which the two electrodes of a bipolar pair are brought sufficiently close, such that the gap between them supports the production of water vapour and direct arcing between the two electrodes. Although the technique provides for a method of arcing during immersion in conductive fluid, the gap is so small that the tissue effect is very limited.

U.S. Patent No. 4,116,198, Roos, describes a further technique of overcoming some of the problems associated with use of bipolar arrangements when immersed in electrically conductive solutions. This technique overcomes the limitations of Reimels by use of a return electrode which is set back from the active electrode such that a more protracted electrical circuit is completed by the solution and direct arcing between the two is avoided. By this means only one electrode of the bipolar pair is required to contact tissue and the electric field is sufficient to include the tissue at the application site. This technique was successfully employed in endoscopic surgical procedures involving transuretheral resection of the prostate gland, as described by Elsasser and Roos in an article entitled 'Concerning an Instrument for Transuretheral Resection without Leakage of a Current', published in the German magazine 'Acta Medico Technica' 1976 vol. 24 No. 4 pages 129-134.

The present applicants' co-pending patent applications relating to electrosurgery describe further improvements over the Roos prior art to control the dimensions of the active electrode relative to a complex interaction between the configuration of the active electrode, the power threshold to establish arcing at the active electrode and control features in the electrosurgical generator component of the system. The commercial embodiments of these inventions are now widely used in the fields of arthroscopic, hysteroscopic and urological surgery.

U.S. Patents Nos. 5,366,443 and 5,697,909 in the names of Eggers and Thapliyal describe an alternative approach using an array of active electrodes which can be selectively activated, or current limited, in order to reduce power dissipation into the electrically conductive fluid.

The above arrangements tend to suffer from a number of problems and technical limitations during use. Carbon tracking is a problem wherein the carbon residue derived from tissue vaporisation forms a conductive track between active and return electrodes. Once established, the track has a negative temperature co-efficient of resistance so that the hotter the carbon becomes, the more conductive it becomes and the more current flows along the track between the electrodes. The temperature developed in the track places huge thermal stresses on the insulator separating the return and active electrodes, which may result in catastrophic failure. Similar failures can occur due to overheating of the electrode assembly without carbon track formation when only portions of the distal tip assembly are in contact with fluid such that current can still flow in the absence of sufficient fluid cooling of the assembly.

In these prior devices, the electric field becomes concentrated in the region representing the shortest conduction path through the fluid medium. The shortest paths occur between the most distal portion of the return electrode and the most proximal region of the active electrode exposed to the fluid. The effects of this are two-fold: firstly, the high current density may cause the return electrode to become "active", particularly when the distal portion of the electrode is only partially immersed in fluid due to the accumulation of gaseous by-products produced during vaporisation; and, secondly, the depth of tissue effect is limited by the concentration of the electric field, particularly when a deeper coagulative effect is desirable.

When the active electrode is partially wetted and partially enveloped in vapour, very high powers are required to sustain what is, in effect, an unstable condition which usually results in intermittent collapse of the vapour pocket and variable surgical performance. The prior art describes certain ratios of the shortest and longest conduction path lengths between the active and return electrodes to reduce this effect. The effect is exacerbated by high fluid flows when the vapour pocket can become quenched, and, once again, high powers are required to overcome this quenching effect. Nevertheless, the surgical performance is variable. The prior art includes techniques to reduce this power threshold of vaporisation in the presence of a fluid flow, but the techniques involved impose some geometric limitations on the electrode assembly which can be difficult to implement in certain fields of application, particularly when active suction or fluid delivery occurs adjacent to, or through, the electrode itself.

It is an aim of this invention to provide an electrosurgical instrument for treating tissue immersed in a conductive fluid, which overcomes at least some of the disadvantages set out above.

According to this invention, there is provided an electrosurgical instrument for treating tissue immersed in an electrically conductive fluid with radiofrequency energy in the frequency range of from 100kHz to 50 MHz, wherein the instrument comprises an elongate shaft configured to be mounted at a proximal end to a handpiece and carrying at its distal end a bipolar electrode assembly which includes: an active electrode with an active electrode zone at a distal end of the active electrode; and a return electrode with a return zone near the active zone; wherein at least one of the active and return zones has an electrically insulating dielectric covering such that in use a radio frequency electrical circuit between the active and return electrodes through the conductive fluid is completed by dielectric coupling through the dielectric covering. The invention also includes an electrosurgery system for treating tissue immersed in an electrically conductive fluid, comprising: a generator for delivering a radio frequency tissue treatment output in the frequency range of from 100 kHz to 50 MHz, and an electrosurgical instrument as described above, the bipolar electrode assembly being connected to the generator. In the preferred instrument, the return zone is adjacent and set back from the active zone in the proximal direction. When the electrode assembly is immersed in a conductive fluid, the electrode with the insulating covering is capacitvely coupled to the fluid, the capacitance of the coupling depending on, inter alia, the thickness of the dielectric covering, its relative dielectric constant (εᵣ), and the area of the electrode.

The invention is typically applicable to wet field electrosurgery, the instrument comprising, for instance, a tubular elongate shaft for insertion into a body cavity and, at the end of the shaft for insertion into the body cavity, an electrode assembly comprising a first conductor at an extreme distal end of the instrument and a second conductor insulated from the first conductor and set back from the distal end, wherein at least the second conductor is encased in an insulative outer dielectric layer.

An electrosurgery system including an instrument such as that described above may include a generator having a pair of output terminals coupled respectively to first and second conductors of the electrode assembly, wherein the frequency of operation of the generator and the construction and materials of the second conductor and the encasing insulative layer are such that when a radio frequency current of 2 amps is delivered to the electrode assembly when immersed in normal saline, the current density at the outer surface if the insulative layer does not exceed 50 mA/mm².

The return zone of the return electrode may be defined by an electric field developed between the active electrode and the return electrode when the electrode assembly is connected to the radio frequency output of the generator. An insulator separates the return electrode from the active electrode; the return zone preferably being coated with a layer of dielectric insulating material wherein, in use, the return zone is insulated from direct electrical contact with the tissue and the electrically conductive fluid so that an electrical circuit is completed between the active and return zones by dielectric coupling through the dielectric insulating material.

The active zone may be coated with a second dielectric insulating material layer so that the active zone is insulated from direct electrical contact with the tissue and the electrically conductive fluid and, in use, a circuit is completed between the active and return electrodes by dielectric coupling also through the second dielectric insulating material.

The active electrode may be in the form of a ceramic body having an internal cavity lined with metal, the ceramic body being made of a dielectric insulating material, wherein, in use, an electrical circuit is completed between the active and return zones by dielectric coupling through the ceramic body. The radio frequency output advantageously interacts with the dielectric insulating material to result in a substantially uniformly distribution of the electric field over the return zone and a corresponding substantially uniform current density over the return zone. In the case of the return zone being coated with a second dielectric insulating material, the radio frequency output interacts with the dielectric insulating material and the second dielectric insulating material to result in a substantially uniformly distribution of the electric field over the active and return zones and a corresponding substantially uniform current density over those zones.

Features of other aspects of the invention and its preferred embodiments are included in the claims.

The properties of the dielectric insulating material, coupled with variations in the electrosurgical output frequency and the combination of one or either or both of the active and return zones being coated in the dielectric material, enable manipulation of the electric field and current density created during use in order to confer significant performance advantages over prior art devices. These advantages can be realised in the electrodes geometrically configured to perform specific procedures on different tissue structures such as structures contained within cavities, lumens, ventricles or other natural body cavities containing an electrically conductive fluid, whether or not such fluid is naturally present or introduced as part of the surgical procedure. Such cavities may also be provided by artificial fluid enclosures or by surgical creation of the cavity. Of specific advantage, the performance of instruments in accordance with the invention can be made less susceptible to overheating and the effects of fluid flow on during use.

The invention will now be described by way of example with reference to the drawings in which:-
Figure 1 is a partially sectioned side view of an electrode assembly of a first preferred embodiment of the invention;
Figure 2 is a partially sectioned side view of a prior art electrode assembly;
Figure 3 is a partially sectioned side view of the electrode assembly of a second preferred embodiment of the invention;
Figure 4 is a side view of an electrode assembly having a dielectric coating with different dielectric permitivities in order to define a return zone;
Figures 5A and 5B are partially sectioned side views and equivalent circuits of further electrode assemblies showing additional techniques for defining a return zone;
Figures 6A and 6B are side views of two further electrode assemblies for instruments in accordance with the invention;
Figure 7 is a graph of an impedance versus power relationship; and
Figure 8 is a partially sectioned side view of a further preferred electrode assembly, the features of which may be combined with the features of any of the other illustrated embodiments.

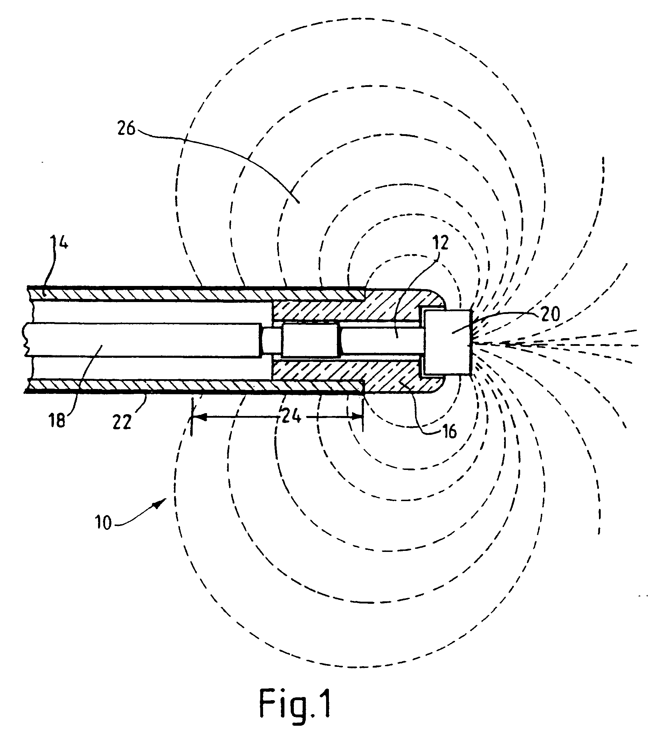

Referring to Figure 1, an electrode assembly 10 is located at the working end of a shaft of an instrument in accordance with the present invention, wherein the return path for the electrosurgical current passing through the electrode assembly is completed by dielectric coupling. The electrode assembly 10 is connected to a generator (not shown) providing a radio frequency output to the assembly in the range of from 100 kHz to 50 MHz. Electrode assembly 10 includes a metallic active electrode 12, a metallic return electrode 14, and a ceramic insulator 16 separating active electrode 12 from return electrode 14. Return electrode 14 is connected to the generator, as is active electrode 12, which is connected to the generator via an active conductor 18 that extends through the hollow centre return electrode 14. Active electrode 12 includes an active zone 20 at the distal end of electrode 12. Return electrode 14 is a metallic conductor, which serves as the shaft of the instrument, and which is covered by a thin plastics dielectric coating 22 over at least a distal end portion, the conductor being completely sealed from the outside. Return electrode 14 also includes a return zone 24 that is substantially at the distal end of electrode 14.

The dielectric material used for coating 22 in the embodiment of Figure 1 preferably has a high relative dielectric constant (e.g. εᵣ in excess of 2) and a low dielectric loss. One material for high frequency use (in excess of 5MHz) is polyimide, which has an exceptional dielectric strength and can be coated in very thin layers. Its relative dielectric constant is 3.4. For example, a 50µm coating of polyimide has sufficient dielectric strength to withstand the typical maximum output voltage of the generator to which electrode assembly 10 is connected. Such voltages may be in the region of 120Vrms for tissue coagulation and 200 to 500Vrms for tissue cutting and vaporisation. It should be noted, however, because the purpose of the dielectric coating is to capacitively couple energy to a conductive solution surrounding electrode 10, it is possible to use dielectric coatings that are thinner than 50µm. For lower frequencies, materials with higher relative dielectric constants are preferred, suitable examples being titanium dioxide (εᵣ=100) and barium titanate compounds (having εᵣ values from 30 to as high as 1600).

The electrode assembly 10 shown in Figure 1 is useful for illustrating the effect of use of a dielectric covering in accordance with the present invention. In the embodiment shown in Figure 1, return electrode 14 has a diameter of 3.3mm. Return zone 24 of return electrode 14 is associated with a distributed electric field pattern 26 that is developed between active electrode 12 and return electrode 14 when such electrodes are connected to the output of the generator to which electrode assembly 10 is attached. Return zone 24 is coated with polyimide dielectric coating 22, and typically extends a distance of at least 10mm, preferably 20mm along shaft 14 from the most distal end of the shaft when the active electrode 12 is not similarly coated. In this case, the overall area of the return zone is, therefore, about 200mm².

Since polyimide has a relative dielectric constant εᵣ of 3.4, the return coupling capacitance to the conductive solution surrounding the electrode assembly 10 is 125pF with a 50µm dielectric coating (or 250pF with a 25µm dielectric coating). At an electrosurgical output frequency of 1MHz this represents an impedance of 1275Ω with a 50µm dielectric coating or 638Ω with a 25µm dielectric coupling. When the electrode assembly is immersed in a conductive solution, such as saline, the conductive path between the active zone 20 and the return zone 24 can have an impedance as low as 50Ω. At such a low impedance compared with that of the coating, very little power would be delivered. However, the impedance of the return coupling capacitance falls with increasing output frequency as shown in Table 1 below.

**TABLE 1**

| Frequency | 50µm coating | 25µm coating |
|---|---|---|
| 6.79MHz | 188Ω | 94Ω |
| 13.56MHz | 94Ω | 47Ω |
| 27.12MHz | 47Ω | 23Ω |
| 40.68MHz | 32Ω | 16Ω |

The above frequencies are in the internationally recognised Industrial Scientific or Medical bands (ISM). Clearly, there are coating thickness and output frequency options for which the majority of the applied voltage will appear across the load rather than the capacitance of dielectric coating 22. There are, however, considerable control implications related to the capacitive coupling effect since it applies to operating in at least two electrosurgical output modes; i.e., one producing tissue desiccation and the other selected levels of tissue vaporisation.

It follows from Table 1 that use of a dielectric coating having εᵣ of the order of that of polyimide, and with the stated thicknesses, requires operation at a relatively high frequency if there is a direct conduction path through conductive fluid between the outer surface of the coating and the surface of the other electrode. In other words, such a coating, when both it and the other electrode are wetted with normal saline, requires delivery of electrosurgical power typically at a frequency of at least 5MHz. It will be appreciated, however, that if the electrosurgical procedure involves vaporisation of a conductive liquid adjacent either surface, the frequency of operation may be switched to a much lower value once vaporisation has been established since the impedance represented by the path between the two surfaces then rises. Indeed, the impedance may rise by a factor of 20 when a vapour pocket is established, which means that the impedance represented by the coating at the lower frequency is much less significant.

This yields the important concept of a system involving a multiple electrode assembly with at least one electrode having a dielectric coating, and operating the system in an upper frequency range for tissue coagulation or desiccation (with the surfaces of the assembly wetted), and in a lower frequency range for tissue cutting or vaporisation (with at least one of the surfaces lying beneath a layer of vapour). To maintain the same ratio between the inter-electrode impedance and the coating impedance when the former rises by 20 times, the frequency may be lowered also by a factor of 20, typically of 1 MHz or lower.

When operation at lower electrosurgical frequencies with a wetted electrode assembly is required, e.g. less than 5MHz down to about 100kHz, the dielectric coating may be formed of one of the higher dielectric constant materials referred to above. These are ceramic materials and are, therefore, better suited to coating thicknesses greater than 50µm, preferably 100µm or higher, depending on the capacitance required and the dielectric constant of the material.

With a material having a relative dielectric constant of 100, forming a 20mm long window around a cylindrical return conductor of 3.3mm diameter and giving a capacitor plate area of about 200mmsq, a 100µm thick coating provides a capacitance of 1.8nF. This yields the return coupling impedances shown in Table 2 below at different frequencies.

**TABLE 2**

| Frequency | 100µm coating | 200µm coating |
|---|---|---|
| 0.5MHz | 180Ω | 360Ω |
| 1MHz | 90Ω | 180Ω |
| 2MHz | 45Ω | 90Ω |

With very high dielectric constants, thicker coatings can be used, to the extent that the electrode assembly in the region of the return zone can be made as a ceramic tube as the main structural member of the assembly, with a metallised internal face.

In certain other co-pending applications of the applicants relating to electrosurgical devices, considerable importance was attributed to peak applied voltage from which certain voltage thresholds could be derived for controlling the desiccation and vaporisation modes. In the present instruments , however, the capacitance of the coating appears as an impedance between the electrodes which increases the voltage seen at the return zone 24 during the higher current delivery used during desiccation. Knowledge of the impedance of the coating at any particular frequency of operation allows an effective peak voltage to be calculated, i.e. the voltage between the active electrode and the outer surface of the coating, providing the current can also be measured. Thus, the effective peak voltage is defined by the vectoral subtraction of the voltage drop across the coating from the voltage between the active and return electrodes

Since the insulative coatings described herein behave in a capacitive manner, the highest voltage drop across them occurs when current is at a maximum. Maximum current corresponds to the largest possible wetted surface with the saline temperature close to 100°C. (Saline has a negative temperature co-efficient of resistance and, therefore, has highest electrical conductivity just below boiling.) This maximum current condition is normally reached during desiccation and normally exceeded for vaporisation. It is, in fact, desirable to control effective peak voltage at this point for maximum desiccation effect, since this also corresponds to maximum power delivery for any electrode configuration, or for any coating.

Depending on electrode configuration, it is possible for the load impedance to fall to very low values in the region of 50Ω. Values of load impedances in this region are more likely to occur with larger electrode configurations wherein the power threshold could be as high as 160W (i.e., an electrode capable of absorbing 160W, while fully wetted). Such an electrode could draw as much as 3.2 amps of current. In the highest impedance combination of Table 1 (6.79MHz with a 50µm coating), the impedance of the dielectric coating is 188Ω. The voltage across the coating would therefore be 602Vrms, or 1701V peak-to-peak, given that, at the identified output frequencies, the waveform would necessarily be sinusoidal. The dielectric strength of polyimide is 22kV/mm. The 50µm coating, therefore, offers a dielectric strength of 1100V or 2200V peak-to-peak. As the electrosurgical output frequency or area of the coating is increased, there is an improved margin due to the lowered impedance. Typical operating frequencies are the 27 MHz and 40 MHz values mentioned above. The coating is typically a cylinder having an axial length of 10 to 50mm. A diameter of 3.3mm yields a coating area in the range of from 100 to 520 sqmm.

At the higher voltage outputs used to establish and maintain a vaporisation mode, the same electrode example would have a load impedance of approximately 10-20 times that seen in the desiccation mode, i.e., 500 to 1000Ω. Once vaporisation is established, power requirements typically fall by 50% of the maximum wetted power demand for a given electrode. Thus, in the above example, maximum current would be in the region of 0.28 to 0.4 amps. The voltage drop across the coating therefore falls to 50V (6.79MHz with 50µm coating).

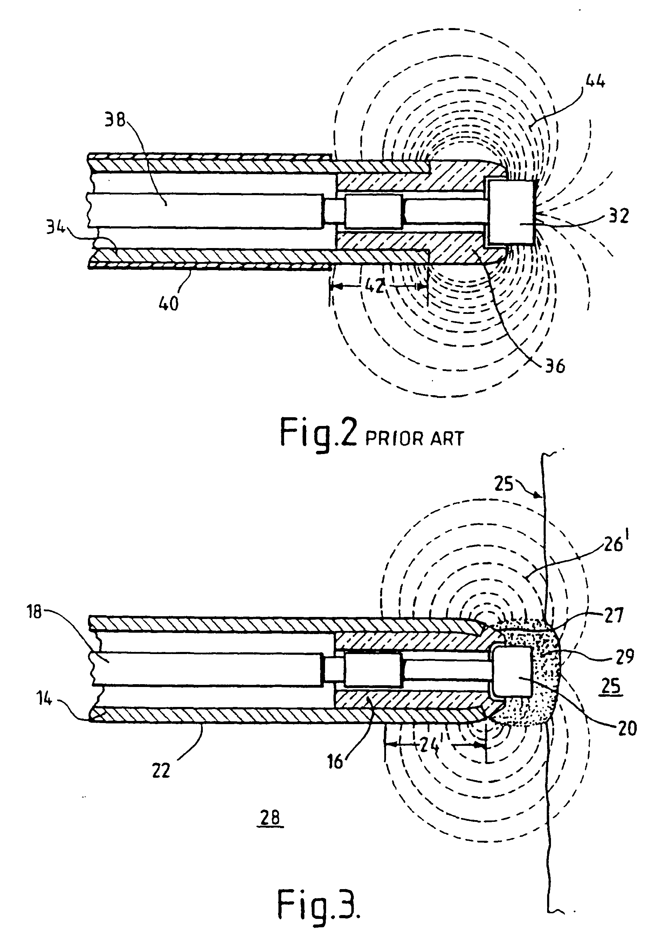

A significant feature of the coating 22 applied over return zone 24 is that power density is limited over the return surface. For example, consider the embodiment in Figure 1 with an output frequency of 13.56MHz and a 50µm dielectric coating, with an applied power of 120W and a load impedance of 50Ω resulting in an applied current of 1.55A. The overall voltage drop across the dielectric return coating 22 is in the region of 145V. Next, consider the shortest conduction path between the active zone 20 and the return zone 24, which can be assumed to be similar to a typical value in prior art devices, such as that shown in Figure 2. The prior art electrode assembly 30 shown in Figure 2 includes a metallic active electrode 32, a metallic return electrode 34, and a ceramic insulator 36 separating active electrode 32 from return electrode 34. Active electrode 32 is connected to a generator via an active conductor 38 which extends through the hollow centre of return electrode 34. Return electrode 34 is also a metallic shaft, which is partially covered by plastics insulation 40, such that an exposed region 42 of return electrode 34 is substantially at the distal end of electrode 34.

The shortest conduction path between the active electrode 32 and the return electrode 34 is in the region of 3mm. However, due to the concentration of current at the distal end of the return electrode 34 as shown by the field pattern lines 44, increasing the size of the electrode (defined by termination of the shaft insulation 40) has little effect on the current density in that region.

The length over which conduction pathways extend proximally along the return zone in the electrode assembly of Figure 1 is very different from that in the example shown in Figure 2. In Figure 1 there is a shortest conduction path of 3mm and a longest of 23mm between the return zone 24 and active zone 20, as compared to 3mm and 6.3mm between the proximal end of the active electrode 32 and the distal and proximal ends of the return electrode 34, respectively. It will be noted from the varying spacing of the field pattern lines 44 that the current density from one end of the return to the other in the prior art example of Figure 2 changes considerably.

In the preferred embodiment of Figure 1, this varying current density causes a varying voltage drop across the dielectric coating 22 to the extent that they are interactive. Where current density tends to be high, the voltage drop across the coating 22 is also high and, conversely, low current density causes a low voltage drop. Thus, the dielectric coating 22 interacts with current so that areas of high current density are reduced and the electric field 26 is more uniformly distributed over a zone of the electrode, the return zone 24. The higher the coating impedance by virtue of thickness or lower output frequency, the more even the current density and the larger is its area of distribution over the return electrode.. This has a significant effect on the electric field 26 produced by the electrode assembly 10 when the active zone 20 is in the uncoated or wetted state. The size of the electric field 26 influences the depth of penetration of the electrosurgical effect into a tissue structure which, with the active zone 20 uncoated or in the wetted state, results in desiccation, coagulation or thermal modification of the tissues.

The exposed conductive return region 42 of the prior art electrode assembly 30 shown in Figure 2 provides no such current density limit. Consequently, there are high current densities at the front edge of exposed region 42 of return electrode 34, which can cause tissue damage if the exposed region 42 is brought into intimate contact with tissue. These high current densities occur even if the length of return electrode exposed region 42 is increased.

A coated return that limits current density, such as return zone 24 shown in Figure 1, can, therefore, be constructed with a larger effective area to disperse current and reduce the propensity for the return to cause tissue damage. Additionally, the limited current density is such that the effects of carbon tracking between the active and return zones, such as zones 20 and 24 of Figure 1, are virtually eliminated.

An additional desirable effect of limiting current density is that the electric field pattern created by the electrode assembly enlarges and becomes more even. Significantly, this enlarged field pattern only occurs in high current situations. Tissue desiccation occurs when the active electrode is wetted, and the impedance is lowest and, therefore, current is the highest. The enlarged field pattern in these circumstances ensures greater current flow in the tissue, and, therefore, increases the desiccation effect for a given power.

Referring now to Figure 3, a modified form of the electrode assembly of Figure 1 has an active electrode 12 used for vaporisation, with the separation from the return zone being 1mm or less. In this case, it is desirable that the field pattern 26' is small so as not to create excessive thermal damage. When used for vaporisation, there is a dramatic load impedance increase of 20 times, maybe up to 50 times, that of the load impedance seen during desiccation. Typically, there is also a decreased power requirement to about 50%. The current can, therefore, fall to 10% of its original value. The voltage drop across the coating 22, therefore, decreases by 90%, and the electric field pattern 26' collapses as a result of the low current densities, as shown in the example of Figure 3. This is highly advantageous because it then minimises the depth of thermal effect. The effective area of the return zone 24' therefore dynamically changes in the different output modes as well as being a function of the properties of the dielectric material 22 and the electrosurgical output frequency.

A small field pattern, such as that shown in Figure 3, during tissue vaporisation is desirable because heating of the saline 28 other than close to the electrode assembly is undesirable. Prior art electrode configurations would normally be limited to a minimum conductive path length to prevent potential breakdowns occurring over the insulator separating the exposed active and return electrodes. Thus, in exposed electrode designs, there is a minimum sized field pattern defined by the minimum conductive path length. Typically, therefore, the conductive pathway of an exposed electrode configuration, such as that shown in Figure 2, extends beyond the field of vaporisation to avoid direct arcing between the two electrodes 32 and 34, which inevitably results in some collateral thermal effects beyond the margin of tissue removal.

If, however, one or other or both return and active zones 24' and 20 are coated with low loss dielectric material, it is possible for the electrode separation zone 27 to fall within the field of vaporisation 29, as shown in Figure 3. Electrical discharge may still occur directly between the regions of the return and active zones 24' and 20 within the vapour pocket 29, but, due to there being no surface charge on the dielectric coating 22, these are low energy discharges with the high energy discharges still preferentially occurring to the tissue surface 25. Thermal dissipation from electrode assembly 10 to surrounding saline, tissue or carbon residues is significantly reduced.

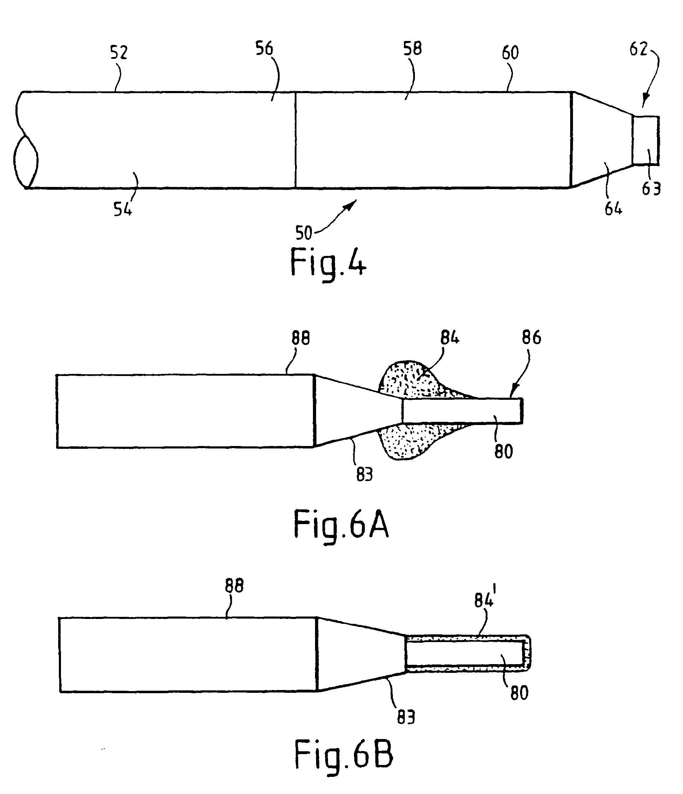

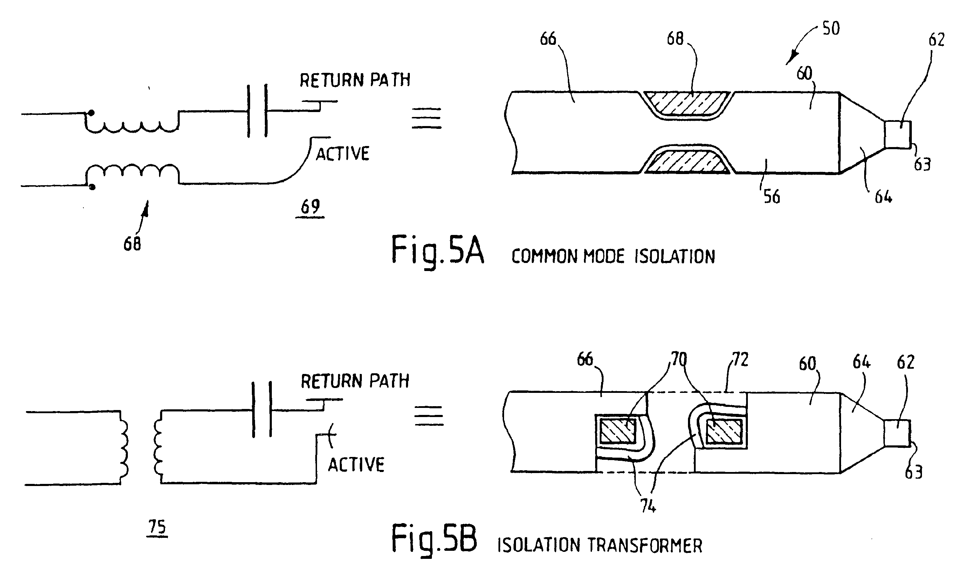

The lower the capacitance between the dielectric coated return to the conductive fluid, the higher is the voltage of the return electrode with respect to the patient potential. As explained above, this voltage drop is advantageous in terms of the effects. Capacitive coupling to other instruments or to tissue at the entry point of the electrode assembly to the body cavity can be prevented by enclosing the shaft proximally of the return zone in a thicker low permitivity dielectric, as shown in Figure 4, or by electrically isolating the tip of the electrode assembly, i.e. including the return electrode, by means of an in-line choke or isolation transformer, as shown in Figure 5.

Referring to Figure 4, an electrode assembly 50 has a thicker dielectric coating 52 of relatively low permittivity applied over a region 54 of a return electrode shaft 56 proximal to a return zone 58 defined by an area of thinner dielectric coating 60 of relatively high permittivity to yield capacitance per unit area to surrounding conductive fluid which is lower than that in the return zone. The electrode assembly 50 shown in Figure 4 also includes an active electrode 62 with an active zone 63 that is separated from return electrode 56 by an insulator 64. The dielectric arrangement shown in Figure 4 further reduces current densities to levels sufficient to prevent tissue damage when in contact with the proximal region 54 of the shaft 56. This arrangement does not, however, prevent capacitive coupling of some energy into a metal object. Such coupling is most likely to occur when the electrode assembly 50 is introduced to the operative site through a metallic channel such as the working channel of an endoscope (not shown). In these circumstances it may be preferable to use one or other of the solutions described with reference to Figures 5A and 5B.

The elevated potential of return electrode 56 can be isolated to the distal end of electrode assembly 50 by using a common mode choke 68 (Figure 5A) or an isolating transformer 70 (Figure 5B). The common mode isolation arrangement of Figure 5A is probably the simpler of the two to realise, since the common mode choke can be constructed as a ferrite toroid or toroidal sleeve 68 around the electrode shaft 56, and, therefore, involves no wound components. For example, a high permeability material can be used to construct a toroidal sleeve with a current-area product AI of 2µH or more. At 40.68MHz this provides an isolation impedance of 500Ω. The potential of isolated shaft 66 with respect to ground is negligible. The return zone 60 is defined by that portion of the insulated shaft distal of the choke 68 which with the active zone 63 is then free to assume any potential with respect to ground. A schematic 69 of the choke arrangement is also shown in Figure 5A. The same outcome provided by the choke arrangement of Figure 5A is also provided by the transformer arrangement of Figure 5B, wherein the high permeability ferrite toroid 70 is utilised as an isolation transformer mounted in a supporting insulator member 72 and including at least single turn windings 74 each in the form of, for instance, a single turn linking the inner active conductor to the surrounding return conductor (forming a coaxial sheath as in the embodiments described above). The proximal isolated shaft 66 is then isolated from the potential developed in the return zone 60 distally of the transformer arrangement. A schematic 75 of the transformer arrangement is also shown in Figure 5B.

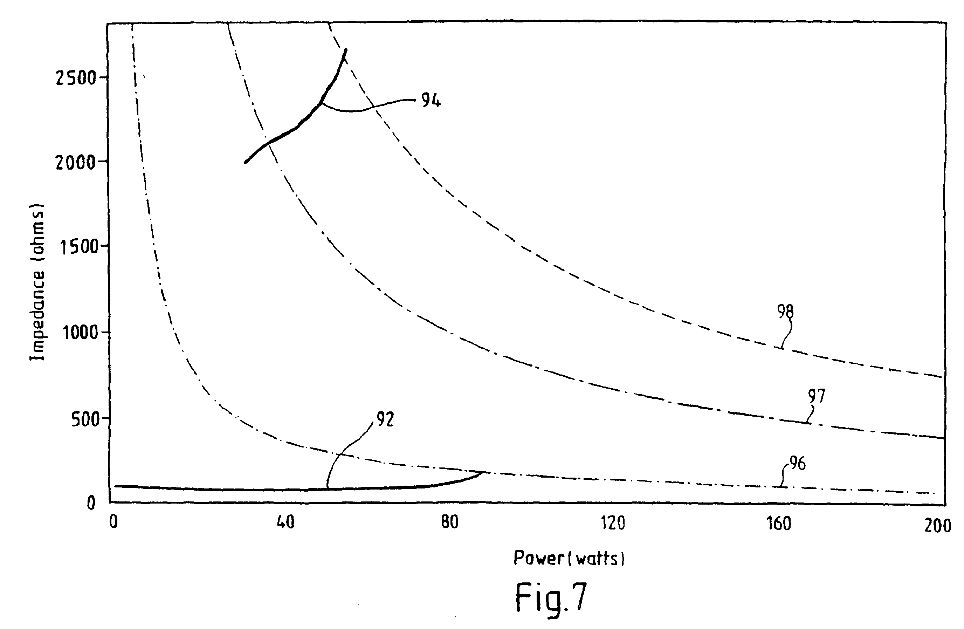

In prior art devices, the area of the active electrode is invariably less than that of the return electrode so as to promote increased power densities at the active electrode. Alternatively, other prior art strategies can be employed to define the active electrode despite the fact that the contact area is at least as large as the return electrode. These strategies are, however, ineffective when the active electrode is configured as a long needle or wire form (due to varying current densities) such as that depicted in Figure 6A. A long thin active electrode 80, as shown in Figure 6A, projecting from an insulator 83 can result in a condition where the most proximal portion is surrounded by a vapour pocket 84, yet the distal end 86 is still in the wetted state. This causes very high power demand due to the high voltage applied to sustain vaporisation simultaneously to a direct saline circuit path. To overcome this situation, the prior art makes use of various geometric limitations related to the longest and shortest conduction path lengths between the active electrode 80 and the most distally disposed edge of the return electrode 88. These limitations, in effect, define the length of the active electrode 80 relative to the current density distribution over the surface of such electrode. Dielectric coating of the active electrode 80 prevents this condition, as shown in Figure 6B.

A dielectric coating 90 on the long active electrode 80 has similar effects to that of dielectric coating the return electrode 88, but also has some additional beneficial effects.

The fact that the active surface is current density limiting prevents the undesirable effects shown in Figure 6A. The electrode assembly can also operate power-efficiently with the active electrode 80 only partly enveloped with a vapour pocket. This results in a lower power threshold to change from wetted to vaporisation states, since only a small amount of saline over the surface of the electrode has to be vaporised to reach a sustainable condition, whereas an exposed active electrode would have to be completely enveloped. The current density limiting also allows a voltage gradient to be established over the surface of the coated active electrode that reduces the voltage drop per unit treatment axis length through the saline. Thus, when in the vaporisation state, the vapour pocket 84' is more uniform in thickness, as shown in Figure 6B.

The ability of the electrode to support vaporisation over only part of its length is also advantageous in adverse conditions, such as high flow environments. An exposed active electrode is vulnerable to these conditions because, if the vapour pocket is swept away from the side of the electrode, the whole vapour pocket is likely to collapse. Thus, coating a long active electrode with a dielectric allows the vapour pocket to operate efficiently over part of the active electrode and can operate to spread the vapour pocket to some extent over the active electrode.

Figure 7 shows the hysteresis in the load impedance versus applied power characteristic for an exposed electrode design in a low flow environment. When one or each electrode is coated, in accordance with the invention, a similar characteristic applies in respect of the conductive path between any point on the outer surface of the coating in one electrode to the outer surface of the other electrode (or, if coated, the outer surface of its coating). The two bold traces show the operating characteristics in the wetted state 92 (the lower trace) and in the vaporisation state 94 (the upper trace). The constant voltage lines 96, 97, 98 represent desiccate, low vaporise and high vaporise voltage respectively. With an exposed active electrode as in Figure 6A, there can be only one voltage over the surface of the active electrode due to the conductive nature of the metallic material. This is not true, however, for a coated active electrode as in Figure 6B. It is possible for one area of the electrode to behave in the wetted state while the other is in vaporisation state. This is possible on a very small scale. A bubble over the surface of the active electrode defines a vaporisation state over the area of the bubble. Thus, when high flow rates are used, the low impedance created on one side of the active electrode quenches the vaporisation state of an uncoated electrode but only partially quenches a coated electrode (*i.e.*, that part most exposed to flow). Instead of considering a single hysteresis diagram for the electrode, the behaviour of a coated electrode is more appropriately modelled by an infinite number of hysteresis diagrams that are virtually independent.

A unique advantage of the capacitively coupled non-contact active electrode described above is the lack of nerve stimulation. When a metal electrode is used for tissue cutting or vaporisation, there is significant nerve stimulation. It is thought that this stimulation arises due to thermionic rectification as a result of the active electrode being heated by arcing. A dielectric coating as described above prevents electron emission and consequently the thermionic effect. Peak currents drawn by arcs are also reduced by virtue of the surface capacitance of the coating.

The coating on the active zone has to withstand both high temperatures and large temperature gradients. Ceramics are materials capable of withstanding this. Similar design factors are employed such as ensuring high dielectric constants and low coating thickness. There are many materials to chose from with relative dielectric constants as high as 100 (titanium dioxide) or 1600 (BaTiO₃) to materials of dielectric strength up to 50kV/mm (quartz, ruby or aluminium nitride).

There are many viable manufacturing techniques such as hollow cavity forms of ceramic with internally inked and fired metallization or plasma sprayed/vapour deposited ceramic on a metal substrate with laser glazing. Coating processes provide the minimum thickness which is in the region of 0.03mm. The tissue treatment area of the electrode of Figure 1 is in the region of 10mm². If this is coated with alumina, the relative dielectric constant is 10. The coupling capacitance is therefore 27pF for alumina or 270pF for titanium dioxide.

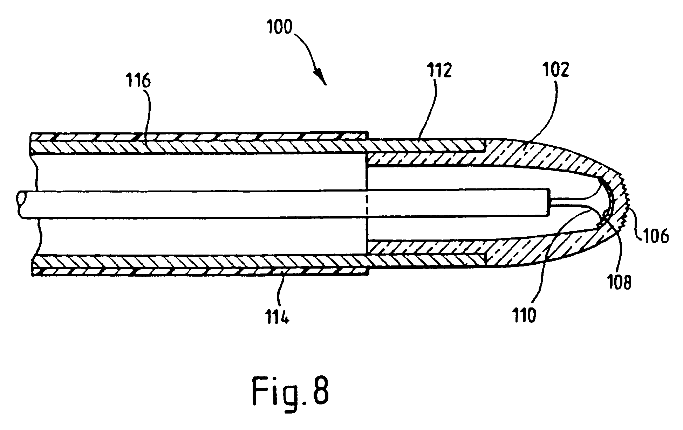

Cavity forms using ceramics are only really viable with high dielectric constant materials. Such a form is illustrated in Figure 8. The electrode assembly 100 shown in Figure 8 includes a hollow ceramic component 102 of thin cross-section. Ceramic nose component 102 has a textured distal end surface 106 which defines the active zone for improved tissue engagement. The internal surface of active zone 106 is coated with a metallised conductive metal layer 108 which is connected by active connection 110 to one output of the electrosurgical generator (not shown). In this example, the return zone 112 is a conductive return with the plastics shaft insulation 114 terminating proximally of the most distal part of the return/shaft 116 of the instrument. Alternatively, the return zone 112 may also be coated with a dielectric material (not shown) so that both the active and return zones 106 and 112 are insulated from the tissue/fluid environment, this being one embodiment wherein all of the above cited advantages may be realised. In providing such an embodiment, the variables of dielectric impedance of both the active zone 106 and return zone 112 must be summated in order to establish the required operating frequency of the electrosurgical generator.

Advantageously, when utilising an embodiment wherein both the active and return zones are insulated by a dielectric material, the dielectric coating of the active can be designed to be imperfect. Dielectric losses at the active surface promote local heating and reduce the power requirement to reach the vaporisation state. Another attraction of this technique is that when vaporisation is attained and the ceramic is subjected to large thermal stress, the dielectric losses are reduced due to the increase in load impedance and the consequent drop in current. BaTiO₃ provides such a lossy dielectric for this application. Other manipulations of the dielectric properties may be advantageous in optimising performance particularly as it relates to the combined dynamic interaction of electrosurgical output mode, output frequency, dielectric properties related to these variables and dielectric temperature.

At normal electrosurgical frequencies, the clinical effects can be attributed entirely to conduction. At higher frequencies, however, dielectric effects of the load have a significant effect. For example, while normal saline (0.9% W/V) has a conductivity of 15.1mS, it has a relative dielectric constant of approximately 100 at the frequencies of interest. At 40.68MHz there is, therefore, a capacitive impedance of 440Ω/cm and a resistive one of 66Ω cm. The capacitive effect within the saline is minimal. However, the major difference is in the electrical behaviour of the target tissue. Cartilage, for example, has a poor conductivity of about 1.5 to 2ms, yet has a similar dielectric constant. Consequently, VHF currents in cartilage are approximately twice those obtained at low frequencies conventially used in electrosurgery, due to dielectric effects. The conductive properties of any high impedance tissue are similarly doubled and thus tissue effects are enhanced.

In summary, significant features of the systems described above include an electrode assembly having at least an active zone for application to the target site and return zone, one or both of the zones being coated with a dielectric material and immersed in a a conductive fluid such that the electrosurgical circuit is completed by capacitive coupling between one or both of the zones and the fluid used to irrigate or distend the operative site. The active zone may be used to vaporise, coagulate, desiccate or thermally modify bodily tissues immersed in the fluid.

The electric field pattern created between the active and return zones of electrode assembly is manipulated using a combination of the dielectric coating thickness for a given dielectric constant, employing a coating material with a variable dielectric constant at different temperatures, and selecting the size and geometry of the return zone of the electrode assembly and the output frequency of the electrosurgical generator.

Uniform current density is provided over the surface of the active zone of the electrode assembly to support and sustain the formation of a partial vapour pocket over one or more sections of the active zone, and wherein the proximity of the return zone to the active zone has minimal influence over the distribution and size of the vapour pocket formed over at least a part of the active zone and further minimises the propensity for collapse of the entire vapour pocket or pockets when the active zone is exposed to a flow of fluid.

Current densities are reduced over one or both of the active and return zones of the electrode assembly such that the currents necessary to elevate the temperature of a carbon track cannot be developed. A further advantage of a wider distribution of current densities over the active and return zones is that the electrosurgical output can only be supported when substantially all of the electrode assembly is immersed, thereby eliminating the overheating encountered during partial immersion of prior art electrode structures.

Such properties may be realised by combination of instruments, application of the specified surgical techniques and identification of such combinations as may be required to apply the invention to different anatomical situations to provide vaporisation, coagulation, desiccation, or to otherwise thermally modify tissue structures.

The foregoing description of preferred embodiments of the invention has been presented for purposes of illustration and description. It is not intended to be exhaustive or to limit the invention to the precise form disclosed, and obviously many modifications, variations and methods of application are possible from the teachings of the examples herein given. The embodiments were chosen to best explain the principles of the invention as they apply to different situations of use and thereby to enable others skilled in the art to best utilise the invention including any modifications as may be necessary to practise additional aspects of use contemplated as a result of this disclosure. It is intended that the scope of the invention be defined by the claims appended hereto.

For the purposes of the law relating to permissible amendments of the present application, it should be noted that, whilst the particular arrangement of the following claims has been prepared with a view to presenting essential and preferred features of the invention in a logical and concise way, we hereby specifically include as part of the content of this application as filed all possible combinations of the individual features contained in the claims or the preceding description, i.e. in addition to presently recited combinations.

## Claims

1. An electrosurgical instrument for treating tissue immersed in an electrically conductive fluid with radio frequency energy in the frequency range of from 100 kHz to 50 MHz, wherein the instrument comprises an elongate shaft configured to be mounted at a proximal end to a handpiece and carrying at its distal end a bipolar electrode assembly which includes:-
an active electrode (12; 62; 80; 108; 110) with an active zone (20; 63; 80; 106) at a distal end of the active electrode; and
a return electrode (14; 56; 88; 116) with a return zone (24; 58; 88; 112) near the active zone;
wherein at least one of the active and return zones has an electrically insulating dielectric covering (22; 60; 90; 102) such that in use a radio frequency electrical circuit between the active and return electrodes through the conductive fluid is completed by dielectric coupling through the dielectric covering.

2. An instrument according to claim 1, wherein the return zone (24; 58; 88; 112) is adjacent and set back from the active zone (20; 63; 80; 106) in the proximal direction.

3. An instrument according to claim 1 or claim 2, wherein the insulating covering (22; 60) encases the return zone (24; 58), and the active zone (20; 63) is exposed.

4. An instrument according to any of claims 1 to 3, wherein the electrode assembly is constructed such that, when an electrosurgical radio frequency current at at least one frequency in the said frequency range is delivered to the assembly when immersed in normal saline, the current density at the outer surface of the dielectric covering (22; 60; 90; 102) does not exceed 50mA/mm².

5. An instrument according to any of claims 1 to 4, wherein the thickness of the or each dielectric covering (22; 60; 90; 102) is greater than 50µm.

6. An instrument according to any of claims 1 to 4, wherein the thickness of the or each dielectric covering (22; 60; 90; 102) is less than 50µm.

7. An instrument according to claim 3, wherein the said dielectric covering (60) which covers the return zone (58) has a first pre-determined thickness and wherein a remainder of the return electrode (56) is coated with a second dielectric covering (52) having a second pre-determined thickness greater than the first pre-determined thickness and capable of substantially preventing capacitive coupling of the said remainder of the return electrode to other instruments or to tissue within a body cavity.

8. An instrument according to claim 7, wherein the return zone is isolated from the said remainder of the return electrode by a common mode choke (68) positioned between the return zone and the said remainder of the return electrode.

9. An instrument according to claim 7, wherein the return zone is isolated from the said remainder of the return electrode by an isolating transformer (70) positioned between the return zone and the said remainder of the return electrode.

10. An instrument according to any of claims 1 to 9, wherein both the active zone and the return zone are encased in respective insulative dielectric coverings, in that the dielectric covering encasing the return zone has a first pre-determined thickness, in that wherein a remainder of the return electrode is coated with a third dielectric insulating material layer having a second pre-determined thickness greater than the first pre-determined thickness and capable of substantially preventing capacitive coupling of the said remainder of the return electrode to other instruments or to tissue within a body cavity.

11. An instrument according to any of claims 1 to 9, wherein the dielectric covering encases the active zone.

12. An instrument according to claim 11, wherein the active electrode is configured as a long needle or wire (80; 110) and wherein the insulating covering on the active zone is of sufficient thickness that current density is limited over the active zone and the active electrode can operate power efficiently when partly enveloped within a vapour pocket during vaporisation.

13. An instrument according to claim 11 or claim 12, wherein the active electrode comprises a ceramic body (102) defining an internal cavity which cavity is lined with metal (108), the ceramic body having an outer tissue or fluid contact surface.

14. An instrument according to any of claims 11 to 13, wherein the return zone is covered with an insulative dielectric outer layer having an outer fluid contact surface.

15. An electrosurgery system for treating tissue immersed in an electrically conductive fluid, comprising:-
a generator for delivering a radio frequency tissue treatment output in the frequency range of from 100 kHz to 50 MHz, and
an electrosurgical instrument according to any preceding claim, the said bipolar electrode assembly being connected to the generator.

16. A system according to claim 15, wherein the frequency of operation of the generator and the construction of the electrode assembly are such that when a radio frequency current of 2 amps is delivered to the electrode assembly when immersed in normal saline, the current density at the outer surface of the dielectric covering does not exceed 50 mA/mm².

17. A system according to claim 15 or claim 16, wherein the frequency of operation of the generator and the construction of the electrode assembly are such that application of radio frequency power by the generator to the electrode assembly results in a substantially uniform distribution of electric field over the said return zone (24; 58; 88; 112) and a corresponding substantially uniform current density of the said electric field.

18. A system according to claim 15, wherein both the active zone and the return zone are encased in respective insulative dielectric coverings so that the said active zone is insulated from direct electrical contact with the tissue and the electrically conductive fluid and, in use, a circuit is completed between the said active and return electrodes by dielectric coupling through both coverings.

19. A system according to claim 18, wherein the frequency of operation of the generator and the construction of the electrode assembly are such that there is a substantially uniformly distribution of electric field over the said active and return zones and a corresponding substantially uniform current density of the said electric field.

20. A system according to any of claims 15 to 19, wherein the material of the or each dielectric covering is a low loss dielectric material.

21. A system according to any preceding claim, wherein the or each said dielectric covering is of sufficient thickness to result in a limited current density over the said active and return zones so as to prevent significant power dissipation in any carbon tracking between the active and return zones during use of the electrode assembly.

## Patentansprüche

1. Elektrochirurgisches Instrument zur Behandlung von Gewebe, das in eine elektrisch leitende Flüssigkeit eintaucht, mit Hochfrequenzenergie im Frequenzbereich von 100 kHz bis 50 MHz, wobei das Instrument einen länglichen Schaft umfasst, der am proximalen Ende zur Anbringung an einem Handstück gestaltet ist und am distalen Ende eine bipolare Elektrodenanordnung trägt, welche umfasst
- eine aktive Elektrode (12, 62, 80, 108, 110) mit einer aktiven Zone (20, 63, 80, 106) am distalen Ende der aktiven Elektrode, und
- eine Rückführungselektrode (14, 56, 88, 116) mit einer Rückführungszone (24, 58, 88, 112) nahe der aktiven Zone,
- wobei zumindest die aktive Zone oder die Rückführungszone eine elektrisch isolierende dielektrische Beschichtung (22, 60, 90, 102) aufweist, so dass beim Gebrauch zwischen der aktiven und der Rückführungselektrode über die leitfähige Flüssigkeit ein Hochfrequenzstromkreis durch dielektrische Kopplung durch die dielektrische Beschichtung hindurch geschlossen wird.

2. Instrument nach Anspruch 1, wobei die Rückführungszone (24, 58, 88, 112) der aktiven Zone (20, 63, 80, 106) benachbart und gegen diese in proximaler Richtung zurückgesetzt ist.

3. Instrument nach Anspruch 1 oder 2, wobei die isolierende Beschichtung (22, 60) die Rückführungszone (24, 58) umschließt und die aktive Zone (20, 63) freiliegt.

4. Instrument nach einem der Ansprüche 1 bis 3, wobei die Elektrodenanordnung so aufgebaut ist, dass die Stromdichte an der Außenfläche der dielektrischen Beschichtung (22, 60, 90, 102) 50 mA/mm² nicht übersteigt, wenn ein elektrochirurgischer Hochfrequenzstrom bei mindestens einer Frequenz im genannten Frequenzbereich an die in physiologische Kochsalzlösung eingetauchte Anordnung abgegeben wird.

5. Instrument nach einem der Ansprüche 1 bis 4, wobei die Dicke der oder jeder dielektrischen Beschichtung (22, 60, 90, 102) größer als 50 µm ist.

6. Instrument nach einem der Ansprüche 1 bis 4, wobei die Dicke der oder jeder dielektrischen Beschichtung (22, 60, 90, 102) kleiner als 50 µm ist.

7. Instrument nach Anspruch 3, wobei die dielektrische Beschichtung (60), welche die Rückführungszone (58) bedeckt, eine erste vorgegebene Dicke hat, und wobei ein Rest der Rückführungselektrode (56) mit einer zweiten dielektrischen Beschichtung (52) beschichtet ist, die eine zweite vorgegebene Dicke größer als die erste vorgegebene Dicke hat und die kapazitive Kopplung des Rests der Rückführungselektrode an andere Instrumente oder an Gewebe in einer Körperhöhle im wesentlichen verhindern kann.

8. Instrument nach Anspruch 7, wobei die Rückführungszone vom Rest der Rückführungselektrode durch eine Gleichtaktdrossel (68) isoliert ist, die zwischen der Rückführungszone und dem Rest der Rückführungselektrode angeordnet ist.

9. Instrument nach Anspruch 7, wobei die Rückführungszone vom Rest der Rückführungselektrode durch einen Trennübertrager (70) isoliert ist, der zwischen der Rückführungszone und dem Rest der Rückführungselektrode angeordnet ist.

10. Instrument nach einem der Ansprüche 1 bis 9, wobei sowohl aktive Zone als auch Rückführungszone in entsprechende isolierende dielektrische Beschichtungen eingeschlossen sind, und wobei die die Rückführungszone einschließende dielektrische Beschichtung eine erste vorgegebene Dicke hat, und wobei ein Rest der Rückführungselektrode mit einer dritten dielektrischen Schicht aus isolierendem Material beschichtet ist, die eine zweite vorgegebene Dicke größer als die erste vorgegebene Dicke hat und die kapazitive Kopplung des Rests der Rückführungselektrode an andere Instrumente oder an Gewebe in einer Körperhöhle im wesentlichen verhindern kann.

11. Instrument nach einem der Ansprüche 1 bis 9, wobei die dielektrische Beschichtung die aktive Zone umschließt.

12. Instrument nach Anspruch 11, wobei die aktive Elektrode als lange Nadel oder langer Draht (80, 110) gestaltet ist, und wobei die isolierende Beschichtung auf der aktiven Zone hinreichend dick ist, damit die Stromdichte über der aktiven Zone begrenzt ist und die aktive Elektrode leistungswirksam arbeiten kann, wenn sie während der Verdampfung teilweise in eine Dampftasche gehüllt ist.

13. Instrument nach Anspruch 11 oder 12, wobei die aktive Elektrode einen keramischen Körper (102) umfaßt, der einen inneren Hohlraum begrenzt, der mit Metall (108) ausgekleidet ist, wobei der keramische Körper eine Außenfläche für den Kontakt mit Gewebe oder Flüssigkeit hat.

14. Instrument nach einem der Ansprüche 11 bis 13, wobei die Rückführungszone mit einer isolierenden dielektrischen Außenschicht bedeckt ist, die eine Außenfläche für den Kontakt mit Flüssigkeit hat.

15. Elektrochirurgisches System zur Behandlung von Gewebe, eintauchend in eine elektrisch leitende Flüssigkeit, umfassend:
- einen Generator zur Abgabe von Hochfrequenzausgangsleistung zur Gewebebehandlung im Frequenzbereich von 100 kHz bis 50 MHz und
- ein elektrochirurgisches Instrument nach einem der vorangehenden Ansprüche, wobei die bipolare Elektrodenanordnung an den Generator angeschlossen ist.

16. System nach Anspruch 15, wobei die Betriebsfrequenz des Generators und der Aufbau der Elektrodenanordnung derart sind, dass die Stromdichte an der Außenfläche der dielektrischen Beschichtung 50 mA/mm² nicht übersteigt, wenn an die in physiologische Kochsalzlösung eintauchende Elektrodenanordnung ein Hochfrequenzstrom von 2 A abgegeben wird.

17. System nach Anspruch 15 oder 16, wobei die Betriebsfrequenz des Generators und der Aufbau der Elektrodenanordnung derart sind, dass die Anwendung von Hochfrequenzleistung durch den Generator auf die Elektrodenanordnung zu einer im wesentlichen gleichmäßigen Verteilung des elektrischen Feldes und zu einer entsprechenden im wesentlichen gleichmäßigen Stromdichte dieses elektrischen Feldes über die Rückführungszone (24, 58, 88, 112) führt.

18. System nach Anspruch 15, wobei sowohl aktive Zone als auch Rückführungszone von entsprechenden isolierenden dielektrischen Beschichtungen umschlossen sind, so dass die aktive Zone von direktem elektrischem Kontakt mit dem Gewebe und der elektrisch leitenden Flüssigkeit isoliert ist und beim Gebrauch durch die dielektrische Kopplung durch beide Beschichtungen hindurch ein Stromkreis zwischen aktiver Elektrode und Rückführungselektrode geschlossen wird.

19. System nach Anspruch 18, wobei die Betriebsfrequenz des Generators und der Aufbau der Elektrodenanordnung derart sind, dass es eine im wesentlichen gleichmäßige Verteilung des elektrischen Feldes über die aktive Zone und die Rückführungszone und eine entsprechende im wesentlichen gleichmäßige Stromdichte dieses elektrischen Feldes gibt.

20. System nach einem der Ansprüche 15 bis 19, wobei das Material der oder jeder dielektrischen Beschichtung ein verlustarmes dielektrisches Material ist.

21. System nach einem der vorangehenden Ansprüche, wobei die oder jede dielektrische Beschichtung eine hinreichende Dicke hat, damit sich eine begrenzte Stromdichte über der aktiven Zone und der Rückführungszone ergibt, um einen wesentlichen Leistungsverlust durch Kohlenstoffspuren zwischen der aktiven Zone und der Rückführungszone beim Gebrauch der Elektrodenanordnung zu verhindern.

## Revendications

1. Instrument d'électrochirurgie pour traiter un tissu plongé dans un liquide électroconducteur avec une énergie en haute fréquence dans la plage de fréquences de 100 kHz à 50 MHz, dans lequel l'instrument comprend un arbre allongé configuré pour être monté sur un manche au niveau d'une extrémité proximale et portant au niveau de son extrémité distale un ensemble d'électrodes bipolaires qui comprend :
une électrode active (12 ; 62 ; 80 ; 108 ; 110) avec une zone active (20 ; 63 ; 80 ; 106) au niveau d'une extrémité distale de l'électrode active ; et
une électrode de retour (14 ; 56 ; 88 ; 116) avec une zone de retour (24 ; 58 ; 88 ; 112) proche de la zone active ;
dans lequel au moins l'une des zones active et de retour a un revêtement diélectrique électriquement isolant (22 ; 60 ; 90 ; 102), de telle sorte qu'en service, on établit par couplage diélectrique au moyen du revêtement diélectrique un circuit électrique haute fréquence entre les électrodes active et de retour à travers le liquide conducteur.

2. Instrument selon la revendication 1, dans lequel la zone de retour (24 ; 58 ; 88 ; 112) est adjacente et en retrait par rapport à la zone active (20 ; 63 ; 80 ; 106) dans la direction proximale.

3. Instrument selon la revendication 1 ou la revendication 2, dans lequel le revêtement isolant (22 ; 60) recouvre la zone de retour (24 ; 58) et la zone active (20 ; 63) est découverte.

4. Instrument selon l'une quelconque des revendications 1 à 3, dans lequel l'ensemble d'électrodes est construit de telle sorte que, lorsqu'on délivre à l'ensemble, lorsqu'il est plongé dans une solution saline normale, un courant électrochirurgical haute fréquence à au moins une fréquence située dans ladite plage de fréquences, la densité de courant sur la surface externe du revêtement diélectrique (22 ; 60 ; 90 ; 102) ne dépasse pas 50 mA/mm².

5. Instrument selon l'une quelconque des revendications 1 à 4, dans lequel l'épaisseur du ou de chaque revêtement diélectrique (22 ; 60 ; 90 ; 102) est supérieure à 50 µm.

6. Instrument selon l'une quelconque des revendications 1 à 4, dans lequel l'épaisseur du ou de chaque revêtement diélectrique (22 ; 60 ; 90 ; 102) est inférieure à 50 µm.

7. Instrument selon la revendication 3, dans lequel ledit revêtement diélectrique (60) qui recouvre la zone de retour (58) a une première épaisseur prédéterminée et dans lequel un reste de l'électrode de retour (56) est recouvert d'un deuxième revêtement diélectrique (52) ayant une seconde épaisseur prédéterminée supérieure à la première épaisseur prédéterminée et qui est capable d'empêcher sensiblement le couplage capacitif dudit reste de l'électrode de retour à d'autres instruments ou à un tissu à l'intérieur d'une cavité corporelle.

8. Instrument selon la revendication 7, dans lequel la zone de retour est isolée à partir dudit reste de l'électrode de retour par une bobine d'arrêt en mode commun (68) placée entre la zone de retour et ledit reste de l'électrode de retour.

9. Instrument selon la revendication 7, dans lequel la zone de retour est isolée dudit reste de l'électrode de retour par un transformateur de séparation (70) placé entre la zone de retour et ledit reste de l'électrode de retour.

10. Instrument selon l'une quelconque des revendications 1 à 9, dans lequel la zone active et la zone de retour sont recouvertes dans des revêtements diélectriques isolants respectifs, dans lequel le revêtement diélectrique recouvrant la zone de retour a une première épaisseur prédéterminée, dans lequel un reste de l'électrode de retour est recouvert d'une troisième couche de matériau isolant diélectrique ayant une seconde épaisseur prédéterminée supérieure à la première épaisseur prédéterminée et capable d'empêcher sensiblement tout couplage capacitif dudit reste de l'électrode de retour à d'autres instruments ou à un tissu à l'intérieur d'une cavité corporelle.

11. Instrument selon l'une quelconque des revendications 1 à 9, dans lequel le revêtement diélectrique recouvre la zone active.

12. Instrument selon la revendication 11, dans lequel l'électrode active est configurée comme une longue aiguille ou un long fil (80 ; 110) et dans lequel le revêtement isolant sur la zone active a une épaisseur suffisante pour que la densité de courant soit limitée sur la zone active et l'électrode active peut générer efficacement de la puissance lorsqu'elle est partiellement enveloppée à l'intérieur d'une poche de vapeur pendant la vaporisation.

13. Instrument selon la revendication 11 ou la revendication 12, dans lequel l'électrode active comprend un corps céramique (102) définissant une cavité interne, laquelle cavité est revêtue de métal (108), le corps céramique ayant une surface de contact avec un tissu ou liquide externe.

14. Instrument selon l'une quelconque des revendications 11 à 13, dans lequel la zone de retour est recouverte d'une couche externe diélectrique isolante ayant une surface de contact avec un liquide externe.

15. Système d'électrochirurgie pour traiter un tissu plongé dans un liquide électroconducteur, comprenant :
un générateur pour délivrer une sortie haute fréquence destinée au traitement de tissus dans la plage de fréquences de 100 kHz à 50 MHz, et
un instrument électrochirurgical selon l'une quelconque des revendications précédentes, ledit ensemble d'électrodes bipolaires étant raccordé au générateur.

16. Système selon la revendication 15, dans lequel la fréquence de fonctionnement du générateur et la construction de l'ensemble d'électrodes sont telles que lorsqu'un courant haute fréquence de 2 ampères est délivré à l'ensemble d'électrodes lorsqu'il est plongé dans une solution saline normale, la densité de courant sur la surface externe du revêtement diélectrique ne dépasse pas 50 mA/mm².

17. Système selon la revendication 15 ou la revendication 16, dans lequel la fréquence de fonctionnement du générateur et la construction de l'ensemble d'électrodes sont telles que l'application d'une puissance haute fréquence par le générateur à l'ensemble d'électrodes entraîne une distribution sensiblement uniforme du champ électrique sur ladite zone de retour (24 ; 58 ; 88 ; 112) et une densité de courant sensiblement uniforme correspondante dudit champ électrique.

18. Système selon la revendication 15, dans lequel la zone active et la zone de retour sont toutes les deux recouvertes dans les revêtements diélectriques isolants respectifs, de sorte que ladite zone active est isolée de tout contact électrique direct avec le tissu et le liquide électroconducteur et, en service, un circuit est établi entre lesdites électrodes active et de retour par couplage diélectrique au moyen des deux revêtements.

19. Système selon la revendication 18, dans lequel la fréquence de fonctionnement du générateur et la construction de l'ensemble d'électrodes sont telles qu'il y a une distribution sensiblement uniforme du champ électrique sur lesdites zones active et de retour et une densité de courant sensiblement uniforme correspondante dudit champ électrique.

20. Système selon l'une quelconque des revendications 15 à 19, dans lequel le matériau du ou de chaque revêtement diélectrique est un matériau diélectrique à faible perte.

21. Système selon l'une quelconque des revendications précédentes, dans lequel le ou chaque dit revêtement diélectrique a une épaisseur suffisante pour entraîner une densité de courant limitée sur lesdites zones active et de retour afin d'empêcher toute dissipation de puissance significative dans n'importe quel cheminement du carbone entre les zones active et de retour lorsqu'on utilise l'ensemble d'électrodes.
